# FASCICULE DE BREVET EUROPEEN

(11) **EP 3 760 262 B1**
(45) Date de publication et mention de la délivrance du brevet: **01.12.2021**
(21) Numéro de dépôt: 20183153.4
(22) Date de dépôt: 30.06.2020
(51) Int. Cl.: A61M 16/00, A61M 16/12, F04D 17/16, F04D 25/08, F04D 27/00, F04D 29/42, H02K 7/14, H02K 11/20

(54) **VENTILATEUR MÉDICAL A CAISSON INTERNE INCLUANT UNE MICRO-SOUFFLANTE MOTORISÉE ET DES CIRCUITS DE GAZ**
MEDIZINISCHES BEATMUNGSGERÄT MIT INNENGEHÄUSE, DAS EIN MOTORISIERTES MIKROGEBLÄSE UND GASKREISLÄUFE UMFASST
MEDICAL VENTILATOR WITH INTERNAL BOX INCLUDING A MOTORISED MICRO-FAN AND GAS CIRCUITS

(30) Priorité: 05.07.2019 FR 1907508
(43) Date de publication de la demande: 06.01.2021
(73) Titulaire: Air Liquide Medical Systems, 92160 Antony (FR)
(72) Inventeur: DAVOINE, Romain, 92160 Antony (FR)
(74) Mandataire: Air Liquide

(56) Documents cités:
- WO-A1-2012/139681
- WO-A1-2013/048238
- DE-A1-102014 009 895
- FR-A1- 2 843 305
- FR-A1- 2 910 079
- FR-A1- 3 019 991
- US-A1- 2012 037 160
- US-A1- 2017 203 063

## Description

L'invention porte sur un appareil d'assistance respiratoire, c'est-à-dire un ventilateur médical permettant de délivrer un flux d'air enrichi en oxygène servant à traiter une ou des pathologies respiratoires chez l'être humain, lequel est équipé d'un caisson multifonction contenant une micro-soufflante motorisée délivrant un gaz respiratoire, tel de l'air ou de l'air enrichi en oxygène, et des circuits de gaz.

Afin d'assister certains patients dans leur fonction respiratoire, on utilise des appareils d'assistance respiratoire, encore appelés « ventilateurs médicaux », délivrant un gaz respiratoire, tel de l'air ou de l'air enrichi en oxygène, à débit non nul et/ou à une pression supérieure à la pression atmosphérique (> 1 atm).

Ces appareils d'assistance respiratoire mettent en œuvre une micro-soufflante motorisée, aussi appelée « compresseur » ou « turbine », servant à aspirer de l'air ambiant et à le délivrer à une pression donnée aux patients. L'aspiration de l'air par la micro-soufflante, pendant le fonctionnement de son moteur, se fait grâce à une ou plusieurs roues à ailettes agencées sur un arbre ou axe rotatif entraîné en rotation par un moteur électrique, la (ou les) roue(s) à ailettes étant mobile(s) en rotation dans le compartiment interne d'une volute.

L'air peut être enrichi en oxygène, c'est-à-dire additionné d'oxygène supplémentaire, notamment lorsque le patient doit recevoir, dans le cadre de son traitement, une proportion d'oxygène supérieure à 21% en volume.

Les documents EP-A-2165078, EP-A-2102504, WO-A-2012/139681, US-A-2008/304986 et WO-A-2013/020167 décrivent des micro-soufflantes de ce type et des appareils d'assistance respiratoire équipés de telles micro-soufflantes.

On connait par ailleurs US-A-2012/037160 qui décrit un appareil d'assistance respiratoire équipé d'un caisson en deux parties incluant un compartiment-moteur avec une micro-soufflante motorisée, des compartiments d'entrée d'air et de sortie de gaz et un circuit d'oxygène.

Par ailleurs, d'autres appareils d'assistance respiratoires sont connus de WO-A-2013/048238, DE-A-102014009895, FR-A-2843305, FR-A-3019991 et US-A-2017/203063.

Or, ce type d'appareil d'assistance respiratoire est souvent encombrant car incluant des circuits de gaz internes complexes, par exemple tortueux, afin d'assurer un bon refroidissement de la micro-soufflante et de satisfaire par ailleurs aux exigences ventilatoires, notamment pour limiter les pertes de charge et assurer de bonnes performances de ventilation et de sécurité pour le patient.

Au vu de cela, le problème qui se pose est donc de pouvoir limiter l'encombrement d'un ventilateur médical, notamment de type urgence, tout en assurant un refroidissement efficace de la micro-soufflante et une compatibilité pneumatique tout en satisfaisant aux exigences de ventilation, i.e. pertes de charge, performances, sécurité patient...

La solution de l'invention porte sur un appareil d'assistance respiratoire, c'est-à-dire un ventilateur médical, comprenant un caisson interne rigide comprenant un compartiment-moteur au sein duquel est agencée une micro-soufflante motorisée, dans lequel le caisson est formé d'un premier et d'un second demi-caisson solidarisés, de manière étanche, l'un à l'autre, ledit caisson rigide comprenant :
- un compartiment d'entrée d'air comprenant une ouverture d'entrée d'air en communication avec l'atmosphère, ledit compartiment d'entrée d'air étant en outre en communication fluidique avec le compartiment-moteur via au moins une ouverture de sortie d'air, i.e. un ou plusieurs orifices de passage de gaz,
- un compartiment de sortie de gaz comprenant une ouverture de fourniture de gaz, ledit compartiment de sortie de gaz étant alimenté en gaz par la micro-soufflante motorisée, typiquement en air ou en air enrichi en O₂, et
- un circuit d'oxygène comprenant une entrée d'oxygène et au moins une sortie d'oxygène, ladite au moins une sortie d'oxygène étant en communication fluidique avec le compartiment-moteur, et
caractérisé en ce que le caisson comprend en outre un circuit de mise à l'air comprenant une entrée d'air aménagée dans le compartiment d'entrée d'air et une sortie d'air, et permettant au patient de pouvoir respirer au travers dudit circuit de mise à l'air en cas de défaillance de la micro-soufflante.

Selon le cas, l'appareil d'assistance respiratoire selon l'invention peut comprendre l'une ou plusieurs des caractéristiques techniques suivantes :
- le premier demi-caisson comprend :
   - au moins une partie du compartiment d'entrée d'air,
   - au moins une partie du compartiment-moteur,
   - au moins une partie du compartiment de sortie de gaz, et
   - le circuit d'oxygène comprenant l'entrée d'oxygène et ladite au moins une sortie d'oxygène.
- le deuxième demi-caisson comprend :
   - au moins une partie du compartiment d'entrée d'air et
   - le circuit de mise à l'air comprenant l'entrée d'air et la sortie d'air.
- le circuit de mise à l'air comprenant une sortie d'air agencée en parallèle de l'ouverture de fourniture de gaz du compartiment de sortie de gaz.
- il comprend des moyens de filtration agencés en amont et/ou en aval du compartiment d'entrée d'air, par exemple un ou plusieurs filtres de type HEPA ou analogue (High Efficiency Particulate Air Filter pour filtre à particules aériennes à haute efficacité), permettant de filtrer l'air ambiant et de le débarrasser des polluants pouvant s'y trouver, tel que poussières, microorganismes, spores...
- le circuit d'oxygène comprend une entrée d'oxygène agencée en parallèle de la sortie d'air du circuit de mise à l'air.
- le compartiment-moteur comprend une paroi périphérique entourant au moins une partie du carter externe de la micro-soufflante, en définissant avec ledit carter externe, un espacement de passage de gaz.
- le circuit d'oxygène comprend au moins une sortie d'oxygène débouchant dans l'espacement de passage de gaz situé entre la paroi périphérique du compartiment-moteur et le carter externe de la micro-soufflante de manière à alimenter ledit espacement du compartiment-moteur avec de l'oxygène et y opérer un mélange dudit oxygène avec de l'air provenant du compartiment d'entrée d'air.
- la micro-soufflante motorisée comprend en outre une volute définissant un compartiment à roue dans lequel est agencé une roue à ailettes portée par un axe-moteur (ou arbre) entraîné par le moteur contenu dans le carter de la micro-soufflante motorisée.
- la volute surmonte le carter du moteur.
- l'axe-moteur se projette dans le compartiment à roue.
- le moteur électrique entraîne en rotation l'axe-moteur portant la roue à ailettes.
- la volute comprend une ouverture de volute en communication fluidique avec, d'une part, l'espacement de passage de gaz et, d'autre part, avec le compartiment à roue de manière à alimenter le compartiment à roue avec du gaz provenant de l'espacement de passage de gaz, ledit gaz étant aspiré dans ledit compartiment à roue par les rotations de la roue à ailettes lorsqu'elle est mise en rotation par le moteur et l'axe qui la porte.
- la volute comprend en outre une ouverture de sortie en communication fluidique avec, d'une part, le compartiment à roue et, d'autre part, avec le compartiment de sortie de gaz de manière à alimenter ledit compartiment de sortie de gaz avec du gaz provenant du compartiment à roue, c'est-à-dire du gaz ressortant du compartiment à roue du fait des rotations de la roue à ailettes. Dit autrement, le gaz est expulsé du compartiment à roue par les rotations de la roue à ailettes lorsqu'elle est mise en rotation par le moteur et l'axe qui la porte.
- le gaz entrant et/ou sortant du compartiment à roue de la volute est de l'air ou de l'air enrichi en oxygène.
- la micro-soufflante est équipée d'un moteur électrique.
- l'oxygène (détendu) introduit dans le compartiment-moteur et qui se mélange à l'air qui s'y trouve, est généralement à une température « froide », c'est-à-dire inférieure à la température ambiante, typiquement 3 à 5°C inférieur, selon le débit délivré, et sa mise en contact avec le carter de la micro-soufflante dans l'espacement de passage de gaz va permettre d'absorber une partie des calories générées par le moteur pendant son fonctionnement (accélération et freinage en ventilation), et le flux de mélange air/oxygène va alors se réchauffer avant d'être envoyé vers la volute.
- la paroi périphérique du compartiment-moteur forme un manchon autour d'au moins une partie du carter externe de la micro-soufflante.
- au moins une partie du mélange gazeux air/oxygène circule dans l'espacement de passage de gaz en balayant la paroi externe du carter de la micro-soufflante.
- le compartiment-moteur comprend en outre au moins une ouverture en communication fluidique avec le compartiment-moteur, en particulier avec l'espacement de passage de gaz, pour extraire au moins une partie du gaz, typiquement de l'air ou un mélange gazeux d'air et d'oxygène, présent dans le compartiment-moteur, en particulier dans l'espacement de passage de gaz.
- le caisson rigide forme une carcasse ou coque.
- il comprend en outre des moyens de pilotage configurés pour commander le fonctionnement ou l'arrêt du moteur électrique, c'est-à-dire les rotations et les arrêts de rotation (i.e. freinage ou décélération) de la roue de la micro-soufflante.
- les moyens de pilotage comprennent au moins un microprocesseur, de préférence au moins un microcontrôleur et des composants d'alimentation du moteur tel des transistors ou autres.
- les moyens de pilotage comprennent au moins une carte électronique portant ledit au moins un microprocesseur et des composants d'alimentation du moteur.
- le microprocesseur met en œuvre un ou plusieurs algorithmes.
- les moyens de pilotage sont alimentés en courant électrique.
- il comprend une IHM.
- il comprend un écran de visualisation, i.e. d'affichage, d'informations.
- les ailettes de la roue à ailettes forment des parois ou structures espacées les unes des autres et agencées radialement sur la première face de la roue à ailettes.
- la roue à ailettes comprend de 10 à 30 ailettes, aussi appelées 'pales'.
- la roue à ailettes a un diamètre compris entre 20 et 80 mm, de préférence entre 30 et 60 mm.
- la roue à ailettes est entraînée par le moteur à une vitesse allant jusqu'à 70 000 tr/min, typiquement jusqu'à 30 000 ou 40 000 tr/min.
- les ailettes forment des parois incurvées, c'est-à-dire non-rectilignes.
- la roue à ailettes est formée d'un matériau polymère, de préférence transformé par injection thermoplastique.
- les ailettes sont formées d'une seule pièce avec la première face de la roue à ailettes.
- le moteur électrique comprend un carter externe formant une structure rigide entourant le rotor et le stator du moteur.
- le moteur électrique comprend des câbles ou fils électriques servant à son raccordement électrique à une source de courant électrique, tel que le secteur ou une (ou des) batterie(s) électrique(s), notamment rechargeable(s).
- la deuxième face de la roue à ailettes, qui fait face au carter du moteur, est dépourvue d'ailettes et est plane.
- le moteur électrique est de type sans balai (« brushless » en anglais).
- la roue à ailettes est agencée mobile en rotation au sein du compartiment à roue de la volute.
- la volute comprend une demi-volute supérieure et une demi-volute inférieure fixée l'une à l'autre, de préférence de manière étanche, par exemple par collage et/ou avec interposition d'un joint d'étanchéité.
- l'axe-moteur portant la roue à ailettes traverse la demi-volute inférieure.
- une ouverture de fourniture de gaz du compartiment de sortie de gaz est connectée fluidiquement à un conduit de fourniture de gaz, tel un tuyau flexible, alimentant une interface respiratoire, tel un masque respiratoire, des canules nasales ou analogue, permettant de distribuer le gaz respiratoire sortant de la micro-soufflante à un patient, typiquement de l'air ou de l'air enrichi en O2.
- le circuit d'oxygène comprenant une entrée d'oxygène alimentée par une source d'oxygène, telle une bouteille d'oxygène sous pression ou une prise murale de distribution d'oxygène alimentée en oxygène par une canalisation d'oxygène, par exemple un réseau hospitalier.

L'appareil d'assistance respiratoire de l'invention est de type à pression continue (CPAP) ou du type à deux niveaux de pression (BiPAP), ou d'urgence.

L'appareil est conçu pour délivrer un flux d'air ou d'air enrichi en oxygène (y compris jusqu'à environ 100% en oxygène).

L'invention porte aussi sur une installation de fourniture de gaz respiratoire à un patient comprenant un appareil d'assistance respiratoire selon l'invention, une source d'oxygène, telle une bouteille d'oxygène sous pression, reliée fluidiquement audit appareil d'assistance respiratoire, un conduit flexible raccordé fluidiquement au et alimenté en gaz par ledit appareil d'assistance respiratoire et une interface respiratoire, telle un masque, des canules nasales ou analogue, alimentée en gaz par ledit conduit flexible, typiquement en air ou mélange air/O₂.

L'invention va maintenant être mieux comprise grâce à la description détaillée suivante, faite à titre illustratif mais non limitatif, en référence aux figures annexées parmi lesquelles :
Fig. 1 représente une vue de côté d'un caisson rigide pour un appareil d'assistance respiratoire selon l'invention,
Fig. 2 est une autre vue de côté du caisson de la Fig. 1,
Fig. 3 est une vue en coupe du caisson de la Fig. 1,
Fig. 4 est une autre vue en coupe du caisson de la Fig. 1,
Fig. 5 est encore une autre vue en coupe du caisson de la Fig. 1,
Fig. 6 est encore une autre vue en coupe du caisson de la Fig. 1,
Fig. 7 est encore une autre vue en coupe du caisson de la Fig. 1, et
Fig. 8 est encore une autre vue en coupe du caisson de la Fig. 1,
Fig. 9 représente une intégration du caisson de la Fig. 1 dans un appareil d'assistance respiratoire.

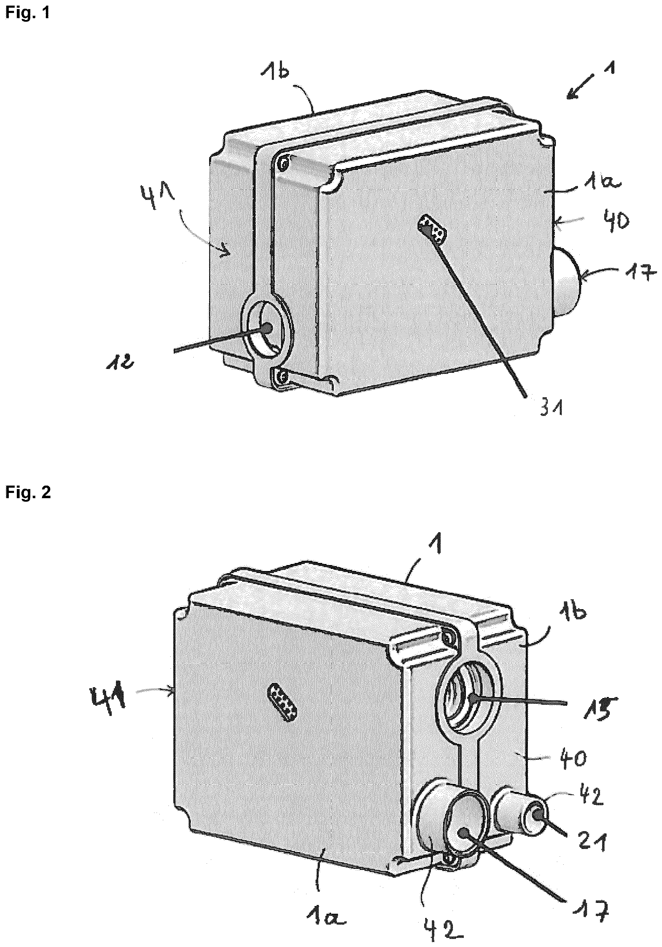

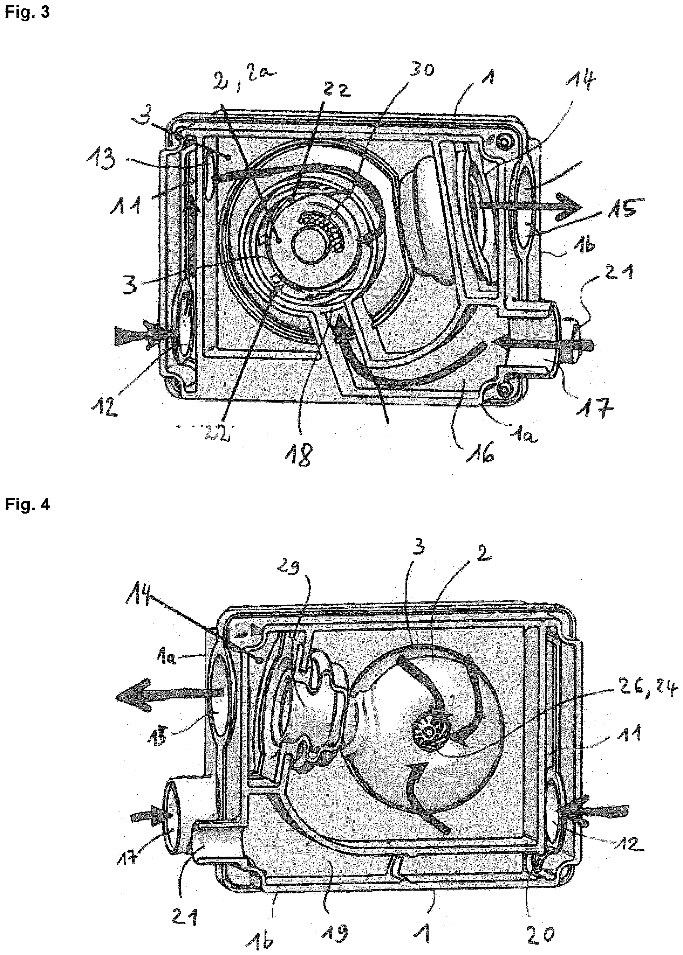

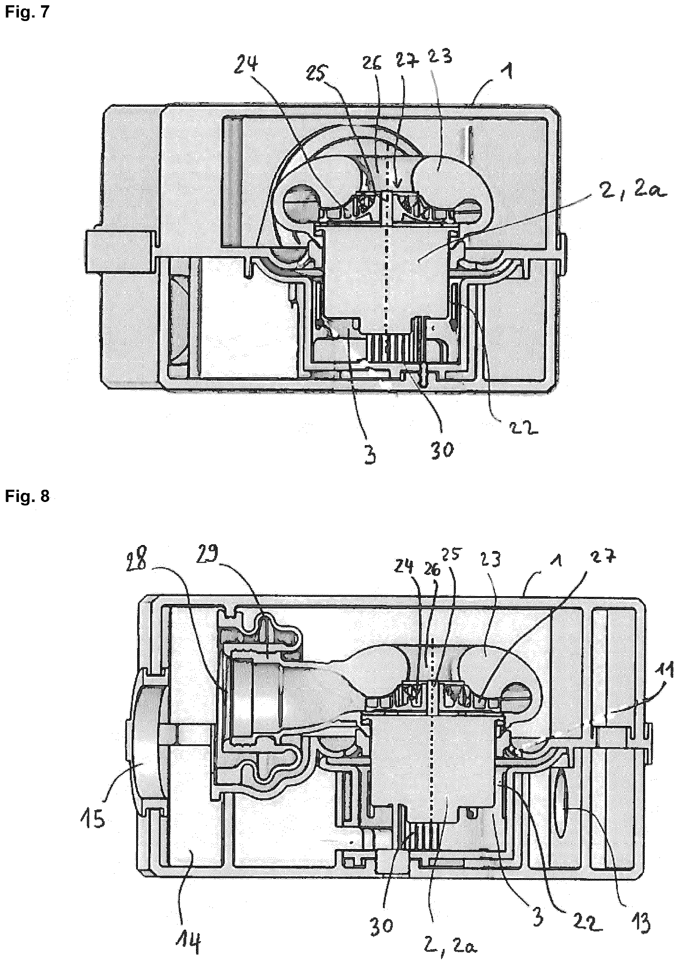

Les Fig. 1 à Fig. 8 représentent un mode de réalisation d'un caisson 1 rigide selon la présente invention pour un appareil d'assistance respiratoire ou ventilateur médical 10 comprenant une micro-soufflante 2 motorisée, encore appelée turbine ou compresseur.

Selon l'invention, le caisson rigide 1 est formé de deux parties, à savoir d'un premier et d'un second demi-caisson 1a, 1b solidarisés, de manière étanche, l'un à l'autre, c'est-à-dire schématiquement à la manière des deux coques ou valves d'un coquillage bivalve.

Dans le mode de réalisation illustré, le caisson 1 a par ailleurs une forme générale parallélépipédique rectangle, comme visible sur Fig. 1. Plus généralement, le caisson rigide 1 comprend :
- un compartiment-moteur 3 au sein duquel est agencée une micro-soufflante motorisée 2,
- un compartiment d'entrée d'air 11 comprenant une ouverture d'entrée d'air 12 en communication fluidique avec l'atmosphère ambiante et par ailleurs avec le compartiment-moteur 3 via une ouverture de sortie d'air 13, de sorte que l'air entrant par l'ouverture d'entrée d'air 12 puisse alimenter le compartiment-moteur 3,
- un compartiment de sortie de gaz 14 comprenant une ouverture de fourniture de gaz 15, lequel est alimenté en gaz par la micro-soufflante motorisée 2 via un conduit d'évacuation 29 de gaz, comme expliqué ci-après,
- un circuit ou passage d'oxygène 16 comprenant une entrée d'oxygène 17 et une ou plusieurs sorties d'oxygène 18, de préférence plusieurs orifices u sorties d'oxygène 18, en communication fluidique avec le compartiment-moteur 3 de manière à alimenter le compartiment-moteur 3 avec de l'oxygène et à y opérer un mélange gazeux entre l'air provenant du compartiment d'entrée d'air 11 et l'oxygène distribué par le ou les sorties d'oxygène 18, et
- un circuit ou passage de mise à l'air 19 comprenant une entrée d'air 20 aménagée dans le compartiment d'entrée d'air 11 et une sortie d'air 21, lequel permet au patient de pouvoir respirer même en cas de défaillance de la micro-soufflante 2 ou analogue.

L'air ambiant peut être filtré avant et/ou après son entrée dans le compartiment d'entrée d'air 11 afin de le purifier, c'est-à-dire de le débarrasser des polluants, tels que poussières, spores, microorganismes ou autres, pouvant s'y trouver, notamment par filtration, par exemple avec un ou des filtres HEPA.

Le premier demi-caisson 1a comprend au moins une partie du compartiment d'entrée d'air 11, au moins une partie du compartiment-moteur 3, au moins une partie du compartiment de sortie de gaz 14, et le circuit d'oxygène (16 avec l'entrée d'oxygène 17 et la ou les sorties d'oxygène 18.

Par ailleurs, le deuxième demi-caisson 1b comprend au moins une partie du compartiment d'entrée d'air 11 et le circuit de mise à l'air 19 avec l'entrée d'air 20 et la sortie d'air 21. Le rôle du circuit de mise à l'air 19 est de permettre à un patient de respirer au travers de ce circuit en cas de défaillance de la micro-soufflante 2, de manière à éviter un accident.

Le circuit ou passage de mise à l'air 19 traverse le caisson et comprend une sortie d'air 21 agencée en parallèle de l'ouverture de fourniture de gaz 15 du compartiment de sortie de gaz 14. De même, le circuit d'oxygène 16 comprend une entrée d'oxygène 17 agencée en parallèle de la sortie d'air 21 du circuit de mise à l'air 19. En d'autres termes, comme visible sur Fig. 2, la sortie d'air 21, l'ouverture de fourniture de gaz 15 et l'entrée d'oxygène 17 sont agencées sur une même face 40 du caisson, alors que l'ouverture d'entrée d'air 12 se situe sur une face opposée 41, comme visible sur Fig. 1.

De préférence, l'entrée d'oxygène 17 et la sortie d'air 21 sont agencées sur des éléments ou raccords tubulaire 42, typiquement cylindrique, faisant saillie en éloignement par rapport à la surface de la face 40 du caisson 1 qui les porte, comme illustré en Fig. 2.

La micro-soufflante 2 motorisée est équipée d'un moteur électrique (non visible), par exemple sans balai (*brushless*), agencé dans un carter 2a externe comprenant une paroi périphérique rigide servant à protéger le moteur, c'est-à-dire typiquement le stator et le rotor du moteur.

La paroi périphérique rigide du carter 2a peut être formée d'un alliage métallique, par exemple un alliage d'aluminium ou du ZAMAK (alliage zinc, aluminium, magnésium et cuivre), ou de polymère ou de thermoplastique, par exemple du PET ou PA. Le carter 2a a généralement une section circulaire, c'est-à-dire une forme générale cylindrique. Il peut aussi comporter des ailettes ou structures de refroidissement faisant office de radiateur.

Le moteur électrique est préférentiellement sans balai ('brushless' en anglais) et/ou est conçu pour atteindre une vitesse de rotation typiquement de l'ordre de 30 000 à 40 000 tr/min, voire jusqu'à 70 000 tr/min ou même au-delà.

L'alimentation en courant électrique du moteur de la micro-soufflante 2 pour permettre son fonctionnement, se fait de manière classique au moyen d'une connectique adaptée, par exemple au moyen d'un ou plusieurs connecteurs, prises 31, fils ou de câbles électriques 30 permettant le raccordement du caisson 1, de l'appareil et/ou de la micro-soufflante 2 au réseau électrique par exemple, ou à une (ou plusieurs) batteries d'alimentation électrique.

Pendant son fonctionnement, le moteur de la micro-soufflante 1 entraîne en rotation, un axe ou arbre 25 rotatif, aussi appelé 'axe-moteur', portant une roue à ailettes 24, aussi appelée 'roue à pales'. Cette roue à ailettes 24 a de préférence une section circulaire de diamètre compris entre 20 et 80 mm, typiquement entre 30 et 60 mm. La roue à ailettes 24 comprend une première face portant les ailettes, aussi appelées 'pales', et une deuxième face dépourvue d'ailettes, c'est-à-dire plane, qui est en outre situé en regard du carter 2a du moteur 2.

La roue à ailettes 24 est agencée de manière à être mobile en rotation au sein du compartiment à roue 27 interne d'une volute 23 surmontant le moteur électrique et le carter 2a, de manière à générer un flux gazeux à une pression supérieure à la pression atmosphérique (> 1 atm).

Préférentiellement, la roue à ailettes 24 et la volute 23 sont en matériau(x) polymère, par exemple un thermoplastique (e.g. PEEK, PA, ...), ou en alliage métallique, par exemple un alliage à base aluminium.

La volute 23 peut être formée de deux demi-volutes, à savoir une demi-volute inférieure et une demi-volute supérieure assemblées et fixées de manière solidaire et étanche l'une à l'autre, par exemple par collage ou autre. Un joint d'étanchéité peut être interposé entre ces demi-volutes.

La volute 23 a une section générale circulaire. Elle comprend en outre une ouverture centrale ou passage de gaz 26 par lequel le gaz est aspiré le compartiment à roue 27 situé dans la volute 23, lors des rotations de la roue 24, et un orifice de sortie de flux de gaz 28 par lequel le flux de gaz généré dans le compartiment à roue 27, lors des rotations de la roue 4, ressort de la micro-soufflante 1.

Le flux gazeux est alors convoyé par un conduit d'évacuation 29 qui est raccordé fluidiquement à l'orifice de sortie de flux de gaz 28, jusqu'à au compartiment de sortie de gaz 14 comprenant une ouverture de fourniture de gaz 15.

En d'autres termes, le compartiment de sortie de gaz 14 est alimenté en gaz par la micro-soufflante 2, via le conduit d'évacuation 29, typiquement en air ou en mélange air/oxygène. Le flux gazeux ressort alors du compartiment de sortie de gaz 14 par l'ouverture de fourniture de gaz 15, avant d'être envoyé ensuite vers le patient, via un tuyau flexible alimentant une interface respiratoire, tel un masque, des canules nasales ou autres (non montrés).

La volute 23 est en communication fluidique, directement ou indirectement, avec le compartiment-moteur 3 au sein duquel s'effectue notamment le mélange air/oxygène lorsque le flux d'air doit être enrichi en oxygène.

Au moins une partie du carter 2a externe du moteur électrique de la micro-soufflante 2 est agencée, c'est-à-dire fait saillie, dans le compartiment-moteur 3 du caisson 1 de l'appareil ventilatoire. Plus précisément, le compartiment-moteur 3 est délimité par (au moins) une paroi périphérique 3a entourant au moins une partie du carter externe 2a de la micro-soufflante 2 en définissant avec le carter externe 2a, un espacement 22 de passage de gaz, au sein duquel peut circuler le flux gazeux, en particulier un mélange air/oxygène, lequel va balayer la paroi du carter 2a avant d'être évacué vers la volute 23. En d'autres termes, la paroi périphérique du compartiment-moteur 3 forme un manchon autour du carter 2a.

Le compartiment-moteur 3 comprend en outre, aménagé dans sa paroi périphérique 3a, un (ou plusieurs) orifice(s) d'entrée d'air 13 en communication fluidique avec l'espacement 22 de passage de gaz pour alimenter le compartiment-moteur 3, y compris l'espacement 22, avec de l'air.

Par ailleurs, il comprend en outre au moins un orifice d'entrée d'oxygène 16, de préférence plusieurs orifices d'entrée d'oxygène 16, en communication fluidique avec le compartiment-moteur 12, y compris l'espacement 22 de passage de gaz, pour alimenter le compartiment-moteur 12 et l'espacement 22 en oxygène et obtenir ainsi un mélange gazeux d'air et d'oxygène, appelé mélange air/oxygène. En fait, une partie du flux gazeux est poussé par le débit d'oxygène entrant dans le caisson.

En d'autres termes, le compartiment-moteur 12, en particulier l'espacement 22, est alimenté à la fois en air du ou des orifices d'entrée d'air 15 et en oxygène provenant du ou des orifices d'entrée d'oxygène 16, ce qui conduit à y réaliser le mélange air/oxygène. Au moins une partie dudit mélange air/oxygène vient au contact du carter 2a de la micro-soufflante 2, c'est-à-dire qu'il vient balayer la surface externe (i.e. paroi) du carter 2a du moteur de la micro-soufflante 2, de sorte de capter une partie des calories (i.e. de la chaleur) générées par le moteur durant son fonctionnement.

Le flux de mélange air/oxygène circulant dans l'espacement 22 s'y réchauffe alors au contact du carter 2, puis sort de l'espacement 22 et donc du compartiment-moteur 12, par une (ou des) ouverture de sortie de gaz 17 mettant en communication fluidique l'espacement 22 de passage de gaz et le compartiment-moteur 12 avec le compartiment à mélange gazeux 18 entourant la volute 5 de manière à extraire au moins une partie du mélange gazeux air/oxygène présent dans l'espacement 22 et qui s'est réchauffé au contact du carter 2 du moteur, et alimenter ensuite le compartiment à mélange gazeux 18 avec ce mélange gazeux air/oxygène.

Ce mélange gazeux air/oxygène est alors aspiré dans le compartiment à roue 6 de la volute 5, via son ouverture centrale ou passage de gaz 7, lors des rotations de la roue 4, c'est-à-dire pendant le fonctionnement du moteur électrique de la micro-soufflante 1 qui entraîne la roue 4 en rotation en créant ainsi une aspiration.

Selon un mode de réalisation, on peut prévoir une chambre ou compartiment intermédiaire entre l'espacement 22 du compartiment-moteur 3 et l'entrée 26 de la volute 23 servant à recueillir l'air ou le mélange gazeux air/oxygène provenant du compartiment-moteur 3 avant qu'il ne pénètre dans la volute 23 en étant aspiré par la roue rotative 24, lors de ses rotations.

Le mélange gazeux air/oxygène ressort ensuite du compartiment à roue 6 de la micro-soufflante 1, via le passage de sortie de flux de gaz 8, avant d'être envoyé ensuite vers un patient qui inhale ce mélange gazeux, par exemple via un tuyau flexible et une interface respiratoire, tel un masque respiratoire ou analogue.

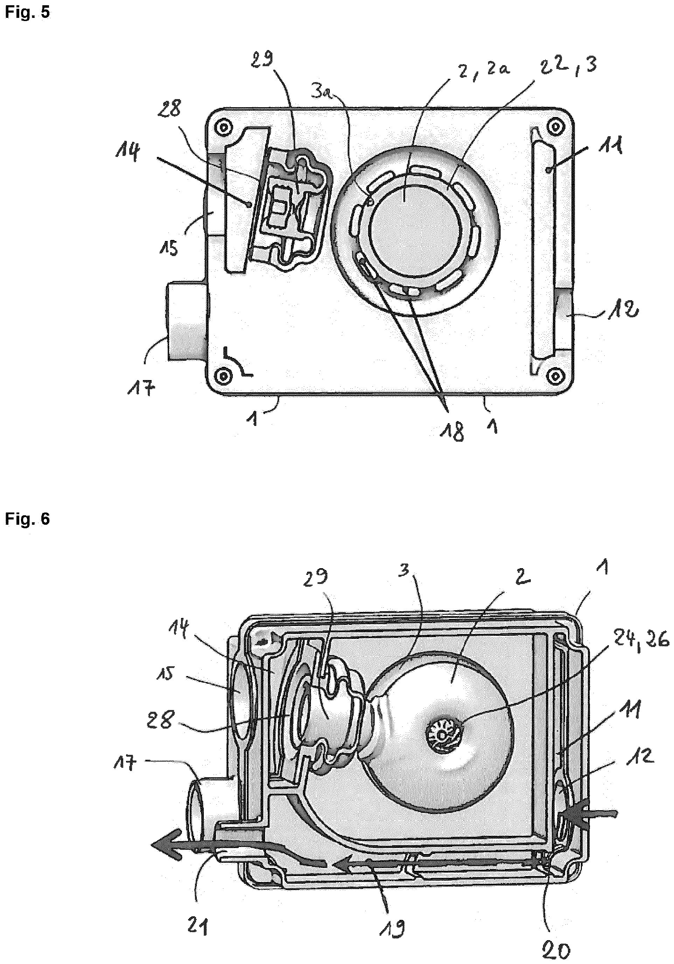

De préférence, l'espacement 22 pour le gaz a une forme générale annulaire, lorsque le carter 2 et le compartiment-moteur 12 ont l'un et l'autre une section circulaire, c'est-à-dire des formes tridimensionnelles générales cylindriques, comme illustré en Fig. 5.

Comme déjà mentionné, le mélange air/oxygène est aspiré par la roue à ailettes 4, durant ses rotations, et circule dans l'espacement 22 en venant balayer, c'est-à-dire « lécher », la surface externe 2a du carter 2 de manière à le refroidir en captant une partie des calories générées par le moteur, durant son fonctionnement.

De façon classique, l'appareil 10 comprend par ailleurs des moyens de pilotage, telle une carte électronique à microprocesseur, configurés pour commander le fonctionnement ou l'arrêt du moteur électrique. Les moyens de pilotage sont alimentés en courant électrique.

En outre, l'appareil 10 peut aussi comprendre d'autres éléments, tel des capteurs de pression et débit, des organes de commande pneumatique, électrovanne, clapet anti-retour et valve, un écran de visualisation, une interface homme-machine (IHM)....

L'entrée d'air 12 du caisson est en communication fluidique avec une source d'air, de préférence l'atmosphère ambiante, alors que l'entrée d'oxygène 21 est en communication fluidique avec une source d'oxygène, telle une bouteille de gaz contenant de l'oxygène ou une prise murale de distribution d'oxygène gazeux alimentée par une canalisation de gaz ou un réseau de canalisations. Le gaz provenant de cette source de gaz peut aussi être filtré pour arrêter d'éventuelles poussières ou autres particules.

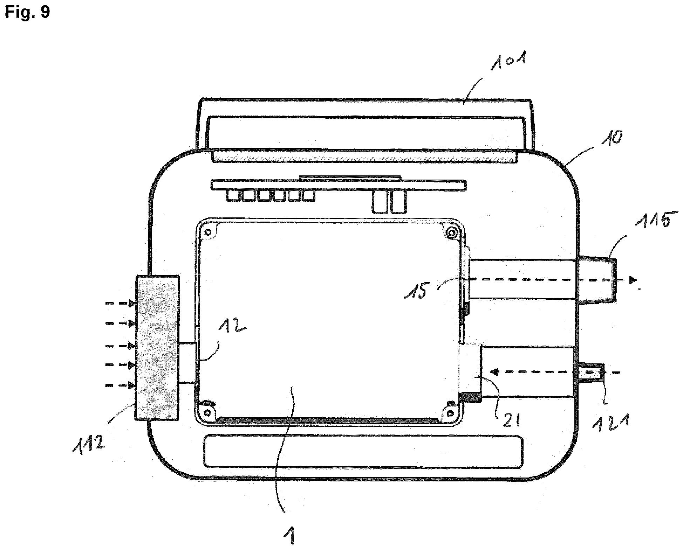

Fig. 9 représente une intégration du caisson 1 de la Fig. 1 dans un appareil d'assistance respiratoire ou ventilateur médical 10, typiquement un appareil délivrant du gaz à un ou plusieurs niveaux de pression, c'est-à-dire un ventilateur de type CPAP ou BiPAP.

Comme on le voit, le ventilateur médical 10 comprend une (ou des) entrée d'air 112 en communication fluidique avec le (ou les) orifice d'entrée d'air 12 du caisson 1, ainsi qu'une entrée d'oxygène 21 en communication fluidique avec le (ou les) orifice d'entrée d'oxygène 21 du caisson. Le ventilateur médical 10 comprend par ailleurs une sortie de flux gazeux 115 en communication fluidique avec le passage de sortie de gaz 15 de la micro-soufflante permettant d'évacuer le mélange air/oxygène pour l'acheminer ensuite vers un patient, via la sortie de distribution de gaz 115 de l'appareil 10.

La ou les entrées d'air 112 sont aménagées dans la carcasse périphérique 102 de l'appareil 10, alors que l'entrée d'oxygène 121 est agencée dans un raccord d'alimentation en oxygène porté par la carcasse de l'appareil 10, relié fluidiquement à une source d'oxygène, telle une bouteille de gaz contenant de l'oxygène ou une prise murale de distribution d'oxygène gazeux alimentée par une canalisation de gaz ou un réseau de canalisations.

Par ailleurs, la carcasse périphérique de l'appareil 10 peut comprendre une poignée 101 de portage facilitant son transport par un utilisateur, comme visible en Fig. 9.

En outre, l'appareil 10 peut aussi comprendre d'autres éléments (non schématisés), tel des capteurs de pression et débit, des organes de commande pneumatique, un écran de visualisation, une interface homme-machine (IHM)...

De façon générale, un appareil d'assistance respiratoire 10 équipé d'un caisson selon l'invention intégrant une micro-soufflante est destiné à traiter des pathologies respiratoires chez des patients humains, adultes et/ou enfants.

## Revendications

1. Appareil d'assistance respiratoire (10) comprenant un caisson (1) rigide comprenant un compartiment-moteur (3) au sein duquel est agencée une micro-soufflante motorisée (2),
dans lequel le caisson (1) est formé d'un premier et d'un second demi-caisson (1a, 1b) solidarisés, de manière étanche, l'un à l'autre, ledit caisson rigide (1) comprenant :
- un compartiment d'entrée d'air (11) comprenant une ouverture d'entrée d'air (12) en communication avec l'atmosphère, ledit compartiment d'entrée d'air (11) étant en outre en communication fluidique avec le compartiment-moteur (3) via au moins une ouverture de sortie d'air (13),
- un compartiment de sortie de gaz (14) comprenant une ouverture de fourniture de gaz (15), ledit compartiment de sortie de gaz (14) étant alimenté en gaz par la micro-soufflante motorisée (2), et
- un circuit d'oxygène (16) comprenant une entrée d'oxygène (17) et au moins une sortie d'oxygène (18), ladite au moins une sortie d'oxygène étant en communication fluidique avec le compartiment-moteur (3), et
**caractérisé en ce que** le caisson (1) comprend en outre un circuit de mise à l'air (19) comprenant une entrée d'air (20) aménagée dans le compartiment d'entrée d'air (11) et une sortie d'air (21), et permettant au patient de pouvoir respirer au travers dudit circuit de mise à l'air (19) en cas de défaillance de la micro-soufflante (2).

2. Appareil selon la revendication 1, **caractérisé en ce que** le premier demi-caisson (1a) comprend :
- au moins une partie du compartiment d'entrée d'air (11),
- au moins une partie du compartiment-moteur (3),
- au moins une partie du compartiment de sortie de gaz (14), et
- le circuit d'oxygène (16) comprenant l'entrée d'oxygène (17) et ladite au moins une sortie d'oxygène (18).

3. Appareil selon l'une des revendications précédentes, **caractérisé en ce que** le deuxième demi-caisson (1b) comprend :
- au moins une partie du compartiment d'entrée d'air (11) et
- le circuit de mise à l'air (19) comprenant l'entrée d'air (20) et la sortie d'air (21).

4. Appareil selon l'une des revendications précédentes, **caractérisé en ce que** le circuit de mise à l'air (19) comprend une sortie d'air (21) agencée en parallèle de l'ouverture de fourniture de gaz (15) du compartiment de sortie de gaz (14).

5. Appareil selon l'une des revendications précédentes, **caractérisé en ce que** le circuit d'oxygène (16) comprend une entrée d'oxygène (17) agencée en parallèle de la sortie d'air (21) du circuit de mise à l'air (19).

6. Appareil selon l'une des revendications précédentes, **caractérisé en ce que** le compartiment-moteur (3) comprend une paroi périphérique (3a) entourant au moins une partie du carter externe (2a) de la micro-soufflante (2), en définissant avec ledit carter externe (2a), un espacement (22) de passage de gaz.

7. Appareil selon la revendication 6, **caractérisé en ce que** le circuit d'oxygène (16) comprend une sortie d'oxygène (18) débouchant dans l'espacement (22) de passage de gaz situé entre la paroi périphérique (3a) du compartiment-moteur (3) et le carter externe (2a) de la micro-soufflante (2) de manière à alimenter ledit espacement (22) du compartiment-moteur (12) avec de l'oxygène et y opérer un mélange dudit oxygène avec de l'air provenant du compartiment d'entrée d'air (11).

8. Appareil selon l'une des revendications 6 ou 7, **caractérisé en ce que** la micro-soufflante motorisée (2) comprend en outre une volute (23) définissant un compartiment à roue (27) dans lequel est agencé une roue à ailettes (24) portée par un axe moteur (25) entraîné par le moteur contenu dans le carter (2a) de la micro-soufflante motorisée (2), ladite volute (23) comprenant une ouverture de volute (26) en communication fluidique avec, d'une part, l'espacement (22) de passage de gaz et, d'autre part, avec le compartiment à roue (27).

9. Appareil selon la revendication 8, **caractérisé en ce que** la volute (23) comprend en outre une ouverture de sortie (28) en communication fluidique avec, d'une part, le compartiment à roue (27) et, d'autre part, avec le compartiment de sortie de gaz (14).

10. Installation de fourniture de gaz respiratoire à un patient comprenant un appareil d'assistance respiratoire selon l'une des revendications précédentes, une source d'oxygène, telle une bouteille d'oxygène sous pression, reliée fluidiquement audit appareil d'assistance respiratoire, un conduit flexible raccordé fluidiquement audit appareil d'assistance respiratoire et alimenté en gaz par ledit appareil d'assistance respiratoire et une interface respiratoire, telle un masque, des canules nasales ou analogue, alimentée en gaz par ledit conduit flexible.

## Patentansprüche

1. Gerät zur Atmungsunterstützung (10), umfassend ein starres Gehäuse (1), das einen Motorraum (3) umfasst, in dem ein motorisiertes Mikrogebläse (2) angeordnet ist,
wobei das Gehäuse (1) aus einer ersten und einer zweiten Gehäusehälfte (1a, 1b) gebildet ist, die dicht miteinander verbunden sind, wobei das starre Gehäuse (1) umfasst:
- einen Lufteinlassraum (11), der eine mit der Außenluft in Verbindung stehende Lufteinlassöffnung (12) umfasst, wobei der Lufteinlassraum (11) ferner über mindestens eine Luftauslassöffnung (13) in Fluidverbindung mit dem Motorraum (3) steht,
- einen Gasauslassraum (14), der eine Gasabgabeöffnung (15) umfasst, wobei der Gasauslassraum (14) von dem motorisierten Mikrogebläse (2) mit Gas versorgt wird, und
- einen Sauerstoffkreis (16), der einen Sauerstoffeinlass (17) und mindestens einen Sauerstoffauslass (18) umfasst, wobei der mindestens eine Sauerstoffauslass in Fluidverbindung mit dem Motorraum (3) steht, und **dadurch gekennzeichnet, dass** das Gehäuse (1) ferner einen Lüftungskreis (19) umfasst, der einen Lufteinlass (20), der im Lufteinlassraum (11) angeordnet ist, und einen Luftauslass (21) umfasst und es dem Patienten ermöglicht, bei einem Ausfall des Mikrogebläses (2) über den Lüftungskreis (19) atmen zu können.

2. Gerät nach Anspruch 1, **dadurch gekennzeichnet, dass** die erste Gehäusehälfte (1a) umfasst:
- mindestens einen Teil des Lufteinlassraums (11),
- mindestens einen Teil des Motorraums (3),
- mindestens einen Teil des Gasauslassraums (14) und
- den Sauerstoffkreis (16), der den Sauerstoffeinlass (17) und den mindestens einen Sauerstoffauslass (18) umfasst.

3. Gerät nach einem der vorhergehenden Ansprüche, **dadurch gekennzeichnet, dass** die zweite Gehäusehälfte (1b) umfasst:
- mindestens einen Teil des Lufteinlassraums (11) und
- den Lüftungskreis (19), der den Lufteinlass (20) und den Luftauslass (21) umfasst.

4. Gerät nach einem der vorhergehenden Ansprüche, **dadurch gekennzeichnet, dass** der Lüftungskreis (19) einen Luftauslass (21) umfasst, der parallel zur Gasabgabeöffnung (15) des Gasauslassraums (14) angeordnet ist.

5. Gerät nach einem der vorhergehenden Ansprüche, **dadurch gekennzeichnet, dass** der Sauerstoffkreis (16) einen Sauerstoffeinlass (17) umfasst, der parallel zum Luftauslass (21) des Lüftungskreises (19) angeordnet ist.

6. Gerät nach einem der vorhergehenden Ansprüche, **dadurch gekennzeichnet, dass** der Motorraum (3) eine Umfangswand (3a) umfasst, die mindestens einen Teil des Außenmantels (2a) des Mikrogebläses (2) umgibt, wobei sie mit dem Außenmantel (2a) einen Gasdurchtrittszwischenraum (22) definiert.

7. Gerät nach Anspruch 6, **dadurch gekennzeichnet, dass** der Sauerstoffkreis (16) einen Sauerstoffauslass (18) umfasst, der in den Gasdurchtrittszwischenraum (22) mündet, der zwischen der Umfangswand (3a) des Motorraums (3) und dem Außenmantel (2a) des Mikrogebläses (2) gelegen ist, so dass der Zwischenraum (22) vom Motorraum (12) mit Sauerstoff versorgt wird und darin ein Vermischen des Sauerstoffs mit der vom Lufteinlassraum (11) kommenden Luft vorgenommen wird.

8. Gerät nach einem der Ansprüche 6 oder 7, **dadurch gekennzeichnet, dass** das motorisierte Mikrogebläse (2) ferner eine Spirale (23) umfasst, die einen Radraum (27) definiert, in dem ein Flügelrad (24) angeordnet ist, das von einer Motorachse (25) getragen wird, die von dem in dem Mantel (2a) des motorisierten Mikrogebläses (2) enthaltenen Motor angetrieben wird, wobei die Spirale (23) eine Spiralenöffnung (26) umfasst, die in Fluidverbindung zum einen mit dem Gasdurchtrittszwischenraum (22) und zum anderen mit dem Radraum (27) steht.

9. Gerät nach Anspruch 8, **dadurch gekennzeichnet, dass** die Spirale (23) ferner eine Auslassöffnung (28) umfasst, die in Fluidverbindung zum einen mit dem Radraum (27) und zum anderen mit dem Gasauslassraum (14) steht.

10. Anlage zur Abgabe von Atemgas an einen Patienten, umfassend ein Gerät zur Atmungsunterstützung nach einem der vorhergehenden Ansprüche, eine Sauerstoffquelle wie eine Sauerstoffdruckflasche, die fluidisch mit dem Gerät zur Atmungsunterstützung verbunden ist, einen Schlauch, der fluidisch an das Gerät zur Atmungsunterstützung angeschlossen ist und von dem Gerät zur Atmungsunterstützung mit Gas versorgt wird, und eine Atemschnittstelle wie eine Maske, Nasenkanülen oder Ähnliches, die von dem Schlauch mit Gas versorgt wird.

## Claims

1. Respiratory assistance apparatus (10) comprising a rigid casing (1) comprising a motor compartment (3) within which a motorized micro-blower (2) is arranged, in which the casing (1) is formed from a first and a second half-casing (1a, 1b) which are rigidly connected to each other in a leaktight manner, said rigid casing (1) comprising:
- an air inlet compartment (11) comprising an air inlet opening (12) in communication with the atmosphere, said air inlet compartment (11) additionally being in fluidic communication with the motor compartment (3) via at least one air outlet opening (13),
- a gas outlet compartment (14) comprising a gas supply opening (15), said gas outlet compartment (14) being fed with gas by the motorized micro-blower (2), and
- an oxygen circuit (16) comprising an oxygen inlet (17) and at least one oxygen outlet (18), said at least one oxygen outlet being in fluidic communication with the motor compartment (3),
**characterized in that** the casing (1) additionally comprises a venting circuit (19) comprising an air inlet (20), formed in the air inlet compartment (11), and an air outlet (21), and allowing the patient to be able to breathe through said venting circuit (19) in the event of a failure of the micro-blower (2).

2. Apparatus according to Claim 1, **characterized in that** the first half-casing (1a) comprises:
- at least part of the air inlet compartment (11),
- at least part of the motor compartment (3),
- at least part of the gas outlet compartment (14), and
- the oxygen circuit (16) comprising the oxygen inlet (17) and said at least one oxygen outlet (18).

3. Apparatus according to either of the preceding claims, **characterized in that** the second half-casing (1b) comprises:
- at least part of the air inlet compartment (11), and
- the venting circuit (19) comprising the air inlet (20) and the air outlet (21).

4. Apparatus according to one of the preceding claims, **characterized in that** the venting circuit (19) comprises an air outlet (21) arranged parallel to the gas supply opening (15) of the gas outlet compartment (14).

5. Apparatus according to one of the preceding claims, **characterized in that** the oxygen circuit (16) comprises an oxygen inlet (17) arranged parallel to the air outlet (21) of the venting circuit (19).

6. Apparatus according to one of the preceding claims, **characterized in that** the motor compartment (3) comprises a peripheral wall (3a) surrounding at least part of the outer housing (2a) of the micro-blower (2), thus defining, with said outer housing (2a), a space (22) for passage of gas.

7. Apparatus according to Claim 6, **characterized in that** the oxygen circuit (16) comprises an oxygen outlet (18) opening into the gas passage space (22) situated between the peripheral wall (3a) of the motor compartment (3) and the outer housing (2a) of the micro-blower (2), so as to feed said space (22) of the motor compartment (12) with oxygen and to mix said oxygen there with the air coming from the air inlet compartment (11).

8. Apparatus according to either of Claims 6 and 7, **characterized in that** the motorized micro-blower (2) additionally comprises a volute (23) defining a wheel compartment (27) which accommodates a bladed wheel (24) supported by a motor shaft (25) driven by the motor contained in the housing (2a) of the motorized micro-blower (2), said volute (23) comprising a volute opening (26) in fluidic communication with, on the one hand, the gas passage space (22) and, on the other hand, the wheel compartment (27).

9. Apparatus according to Claim 8, **characterized in that** the volute (23) additionally comprises an outlet opening (28) in fluidic communication with, one the one hand, the wheel compartment (27) and, on the other hand, the gas outlet compartment (14).

10. Installation for supplying respiratory gas to a patient, comprising a respiratory assistance apparatus according to one of the preceding claims, an oxygen source, such as a pressurized oxygen canister, connected fluidically to said respiratory assistance apparatus, a flexible conduit connected fluidically to said respiratory assistance apparatus and fed with gas by said respiratory assistance apparatus, and a respiratory interface, such as a mask, nasal cannulas or the like, fed with gas by said flexible conduit.
